# NEW EUROPEAN PATENT SPECIFICATION

(11) **EP 0 538 904 B2**
(45) Date of publication and mention of the opposition decision: **10.10.2001**
(45) Mention of the grant of the patent: 17.12.1997
(21) Application number: 92118266.3
(22) Date of filing: 26.10.1992
(51) Int. Cl.: C08B 37/00, C08B 11/20, C08B 15/10, A61L 15/00

(54) **Carboxyalkyl polysaccharides and process for their preparation**
Carboxyalkylpolysaccharide und Verfahren zu ihrer Herstellung
Carboxyalkylpolysaccharides et procédé pour les préparer

(30) Priority: 25.10.1991 US 782853; 11.12.1991 US 808086
(43) Date of publication of application: 28.04.1993
(73) Proprietor: KIMBERLY-CLARK WORLDWIDE, INC., Neenah, Wisconsin 54956 (US)
(72) Inventor: Ning, Xin, Appleton, Wisconsin 54914 (US); Sun, Tong, Neenah, Wisconsin 54956 (US)
(74) Representative: Diehl, Hermann O. Th., Dr.

(56) References cited:
- EP-A- 0 201 895
- EP-B- 0 201 895
- EP-B- 0 566 118
- WO-A-90/10495
- DD-A- 212 969
- FR-A- 2 510 628
- GB-A- 1 086 323
- GB-A- 1 397 154
- US-A- 3 723 413
- US-A- 3 731 686
- US-A- 3 858 585
- US-A- 4 090 013
- US-A- 4 200 736
- Ullmann's Encyclopedia of Industrial Chemistry, VCH Verlagsgesellschaft mbH, Weinheim, Germany, 1986, p. 477-479

## Description

The present invention relates to carboxyalkyl polysaccharides having improved absorbent properties. Specifically the present invention relates to carboxyalkyl polysaccharides having the ability to absorb liquid while under a load, and a process for the preparation thereof.

The use of absorbent materials, commonly known as superabsorbents, in disposable absorbent personal care products is known. Such absorbent materials are generally employed in absorbent products such as diapers, training pants, adult incontinence products, feminine care products, and the like, in order to increase the absorbent capacity of such products while reducing their overall bulk. Such absorbent materials are generally present in absorbent products in a fibrous matrix, such as a matrix of wood pulp fluff. A matrix of wood pulp fluff generally has an absorbent capacity of about 6 grams of liquid per gram of fluff. The absorbent materials described above generally have an absorbent capacity of at least about 10, preferably of about 20, and often of up to 100 times their weight in water. Clearly, incorporation of such absorbent materials in personal care products can reduce the overall bulk while increasing the absorbent capacity of such products.

A wide variety of materials have been described for use as absorbent materials in such personal, care products.

Such materials include natural-based materials such as agar, pectin, gums, carboxyalkyl starch, carboxyalkyl cellulose. and the like, as well as synthetic materials such as polyacrylates, polyacrylamides, hydrolyzed polyacrylonitrile, and the like. While the natural-based, absorbent materials are known for use in personal care products, they have not gained wide usage in such products. The natural-based, absorbent materials have not gained wide usage in personal care products, at least in part, because their absorbent properties are inferior compared to the synthetic absorbent materials such as the polyacrylates. Specifically, many of the natural-based materials tend to form soft, gelatinous masses when swollen with a liquid. When employed in absorbent products, the presence of such soft gelatinous masses tends to prevent the transport of liquid within the fibrous matrix in which the absorbent materials are incorporated. This phenomenon is known as gel-blocking. Once gel-blocking occurs, subsequent insults of liquid cannot be efficiently absorbed by the product, and the product tends to leak. Further, many of the natural-based materials exhibit poor absorption properties, particularly when subjected to external pressures.

In contrast, the synthetic, absorbent materials are often capable of absorbing large quantities of liquid while maintaining a generally stiff, non-gelatinous character. Accordingly, the synthetic, absorbent materials can be incorporated in absorbent products while minimizing the likelihood of gel-blocking.

Carboxyalkyl cellulose materials and other carboxyalkyl polysaccharides are known in the art. As a general rule, carboxyalkyl cellulose materials are formed from a cellulosic material which has been treated with carboxyalkylating reactants such as a chloroalkanoic acid, preferably monochloroacetic acid, and an alkali, such as sodium hydroxide, optionally, in the presence of an alcohol. Such a process is described, for example, in U.S. Patent 3,723,413, issued March 27, 1973, to Chatterjee et al. Such carboxyalkyl celluloses are generally water-soluble. Various methods of rendering such water-soluble carboxyalkyl celluloses water-insoluble are known.

U.S. Patent 2,639,239 issued May 19,1953, to Elliott describes a process in which a commercially available water-soluble, alkali-metal salt of carboxymethyl cellulose having a degree of substitution of from about 0.5 to about 1 is subjected to a thermal treatment for up to 10 hours which renders such water-soluble carboxymethyl cellulose capable of forming highly swollen gel particles.

Similarly, U.S. Patent 3,723,413, discussed above, describes the heat treatment of a carboxyalkyl cellulose in the presence of remaining carboxyalkylating reactants and by-products, such that the carboxyalkyl cellulose becomes water-insoluble and possessed of desirable liquid absorptive and retentive properties and characteristics.

U.S. Patent 3,379,720 issued April 23, 1968, to Reid describes a process of preparing modified polysaccharides such as ethers and esters of cellulose comprising slurrying a water-soluble polysaccharide in any inert medium, acidifying said polysaccharide, removing excess acid from the acidified polysaccharide, drying same and heat-curing.

U.S. Patent 4,689,408 issued August 25, 1987, to Gelman et al. describes a method of preparing salts of carboxymethyl cellulose. The method involves treating a carboxymethyl cellulose with water, adding a nonsolvent for the carboxymethyl cellulose, and recovering the carboxymethyl cellulose. The carboxymethyl cellulose is said to have an absorbency of at least 25 grams of liquid per gram of carboxymethyl cellulose.

Unfortunately, the known carboxyalkyl polysaccharide materials do not possess absorptive properties comparable to many of the synthetic, highly absorptive materials. This has prevented widespread use of such carboxyalkyl polysaccharides in absorbent personal care products.

It is desirable to develop and produce a natural-based, highly absorbent material having absorptive properties similar to the synthetic, highly absorptive materials and thus suitable for use in personal care absorbent products.

The present invention concerns a method for producing a waterswellable, water-insoluble carboxyalkyl polysaccharide. The method is characterized by the following steps:
forming a solution consisting of a water-soluble carboxyalkyl polysaccharide and water as the solvent and optionally an acid wherein said solution is acidic or neutral, wherein said water-soluble carboxyalkyl polysaccharide dissolves into said water, said carboxyalkyl polysaccharide having an average degree of substitution from 0.3 to 1.5;
recovering said carboxyalkyl polysaccharide from said solution by evaporative drying; and
heat-treating said recovered carboxyalkyl polysaccharide at a temperature of from 120°C to 200°C and for a time of from 1 min. to 120 min. to crosslink said carboxyalkyl polysaccharide to render said carboxyalkyl polysaccharide water insoluble, wherein said water-insoluble carboxyalkyl polysaccharide has an Absorbency Under Load, as defined in the description, of at least 17 (grams/gram) and a Free-Swell Capacity, as defined in the description, of at least 20 (grams/gram).

Fig. 1 illustrates the apparatus for determining the Absorbency Under Load values of an absorbent material.

Figs. 2-10 illustrate, in the form of graphs, the results of the physical property testing set forth in Tables 1-5.

In one aspect, the present invention concerns a method for producing a water-swellable, water-insoluble carboxyalkyl polysaccharide. The method comprises the steps of forming a solution consisting of carboxyalkyl polysaccharide and water and optionally an acid. The carboxyalkyl polysaccharide is recovered from the solution and heat-treated for a time and at a temperature sufficient to crosslink the carboxyalkyl polysaccharide.

Suitable carboxyalkyl polysaccharides for use in the present invention include carboxyalkyl cellulose such as carboxymethyl cellulose, carboxyethyl cellulose, carboxyalkyl carageenan, carboxyalkyl agar, carboxyalkyl gellan gum and mixtures thereof. The preferred carboxyalkyl polysaccharide is a carboxyalkyl cellulose with the preferred carboxyalkyl cellulose being carboxymethyl cellulose. While any carboxyalkyl polysaccharide is believed suitable for use in the present invention, carboxyalkyl cellulose is preferred. Accordingly, the preferred embodiments discussed below will be described in the context of using carboxyalkyl cellulose as the carboxyalkyl polysaccharide. However, it is to be understood that other suitable carboxyalkyl polysaccharides can be used.

Methods of making carboxyalkyl cellulose are known to those skilled in the art. Suitably a cellulosic material such as wood pulp fluff, cotton, cotton linters, and the like are provided. The cellulosic material may be in the form of fibers or of fibers which have been comminuted to particulate form. The cellulosic material is dispersed in an inert solvent such as an alcohol and carboxyalkylating reagents added to the dispersion. Carboxyalkylating reagents generally comprise a chloroalkanoic acid such as monochloroacetic acid and sodium hydroxide.

It is to be understood that it may be possible to perform the carboxyalkylation of the starting polysaccharide in such a manner that the solution of carboxyalkyl cellulose and water is formed directly. That is, the carboxyalkylation process may be performed in an aqueous medium such that, upon formation of the carboxyalkyl cellulose, it is solubilized in the water. In this manner, no recovery step is necessary between formation of the carboxyalkyl cellulose and the formation of the solution of carboxyalkyl cellulose and water.

The carboxyalkyl celluloses suitable for use in the present invention generally have an average degree of substitution from about 0.3 to about 1.5, preferably from 0.4 to 1.2. The degree of substitution refers to the average number of carboxyl groups present on the anhydroglucose unit of the cellulosic material. When the carboxyalkyl celluloses have an average degree of substitution within the range of from 0.3 to 1.5, the carboxyalkyl cellulose is generally water-soluble.

As used herein, a carboxyalkyl cellulose will be considered to be water-soluble when it dissolves in water to form a true solution.

Carboxyalkyl cellulose is available in a wide range of molecular weights. Carboxyalkyl cellulose having a relatively high molecular weight is desired for use in the present invention. It is generally most convenient to express the molecular weight of a carboxyalkyl cellulose in terms of its viscosity in a 2.0 weight percent aqueous solution. Carboxymethyl celluloses suitable for use in the present invention will generally have a viscosity in a 2.0 weight percent aqueous solution of from about 50 mPa·s (CP) to about 80,000 mPa·s, preferably from about 2,000 to about 80,000 mPa·s, and most preferably from about 20,000 to about 80,000 mPa·s.

Suitable carboxyalkyl celluloses are commercially available from numerous vendors. Exemplary of a commercially available carboxyalkyl cellulose is carboxymethyl cellulose, commercially available from Aqualon Company under the trade designation Aqualon™ or Blanose™ Cellulose Gum.

The solution of carboxyalkyl cellulose and water suitably comprises from 0.01 to 90 weight percent, beneficially from 0.01 to 30 weight percent, and preferably from 2 to 25 weight percent of carboxyalkyl cellulose based on total solution weight. The carboxyalkyl cellulose is suitably dissolved in

The solution of carboxyalkyl cellulose and water and the recovered carboxyalkyl cellulose may be acidic, or neutral. As used herein, acidity will be stated in terms of the degree of molar acidification (DA). The degree of molar acidification is defined as the number of free-acid, carboxyl groups divided by the total number of carboxyl groups, either free-acid or salt-form. The degree of molar acidification is suitably less than 0.07, preferably less than 0.05, assuming use of an essentially completely neutralized carboxyalkyl cellulose having few free acid groups and little, if any, residual base. The solution of carboxyalkyl cellulose and water can be acidified by the addition of an aqueous solution of an inorganic acid such as hydrochloric acid, nitric acid, or an aqueous solution of an organic acid, such as acetic acid.

The solution of carboxyalkyl cellulose and water will suitably have a pH within the range of from 5.0 to neutral beneficially from 6.0 and preferably from 6.5 to neutral. The recovered carboxyalkyl cellulose will generally have the same pH as the solution.

When the carboxyalkyl cellulose of the present invention is intended for use in personal care products such as diapers, training pants, feminine care products, and the like, it is generally desired that the carboxyalkyl cellulose have a generally neutral character. For this reason, it is generally preferred that the solution of carboxyalkyl cellulose and water be formed with a generally neutral pH. Alternatively, if the solution of carboxyalkyl cellulose and water is formed with an acidic pH, the recovered carboxyalkyl cellulose may be neutralized. For example, if the solution is acidic, the recovered carboxyalkyl cellulose will be acidic. The recovered carboxyalkyl cellulose may be neutralized, for example, by contacting with a gaseous base such as ammonia.

The solution of carboxyalkyl cellulose and water can be formed at any temperature at which the carboxyalkyl cellulose is soluble in the water. Generally, such temperatures will be within the range of from about 10°C to about 100°C. As a general rule, it is preferred to form the solution of carboxyalkyl cellulose with agitation.

After forming the solution of carboxyalkyl cellulose and water, the carboxyalkyl cellulose is recovered from the solution by evaporative drying.

As a general rule, the carboxyalkyl cellulose can be recovered by evaporative drying at a temperature within the range from of about 10°C to about 100°C, preferably from about 50°C to about 80°C. Naturally, higher temperatures can be employed if the solution is placed under pressure. Lower temperatures can be employed if the solution is placed under a vacuum.

Depending on the form in which the carboxyalkyl cellulose is recovered, it may be necessary or desirable to alter the form of the carboxyalkyl cellulose. The carboxyalkyl cellulose may be recovered in the form of a film or sheet. It may be desirable to comminute the film or sheet material into particles or flakes of material.

The form of the carboxyalkyl cellulose desired will depend to a large extent on the use for which it is intended. When the carboxyalkyl cellulose is intended for use in absorbent personal care products, it is generally desired that the carboxyalkyl cellulose be in the form of a discrete particle, fiber or flake. When in the form of a particle, it is generally desired that the particle have a maximum cross-sectional diameter within the range from about 50 µm to about 2,000 µm, preferably within the range from about 100 µm to about 1,000 µm, most preferably within the range from about 300 µm to about 600 µm.

The recovered carboxyalkyl cellulose is then heat-treated at from 120°C to 200°C, and preferably from 130°C to 170°C.

The higher the temperature employed, the shorter the period of time necessary to achieve the desired degree of crosslinking. The heat-treating process will extend over a time period within the range of from 1 to 120 min, and preferably from 5 to 60 min.

The inventors found that, by providing the solution of carboxyalkyl cellulose and water with an acidic character the time necessary to effect the heat-treatment can be shortened. Nonetheless, similar general absorptive properties can be achieved with either an acidic, or neutral, carboxyalkyl cellulose. In some instances, it may be desired to provide the solution of carboxyalkyl cellulose and the recovered carboxyalkyl cellulose with an acidic character in order to lower the temperature or shorten the time of the heat treatment. In this instance, the carboxyalkyl cellulose is desirably neutralized after the heat-treatment step.

The heat-treating process causes the carboxyalkyl cellulose to cross link and become water-insoluble. The heat-treating process desirably produces a carboxyalkyl cellulose having the ability to absorb a liquid while the carboxyalkyl cellulose is under a load. Synthetic polymeric materials, such as polyacrylates, having a generally high ability to absorb while under a load have been found to minimize the occurrence of gel-blocking when incorporated in absorbent products. The method by which the Absorbency Under Load is determined is set forth below in connection with the examples. The Absorbency Under Load values determined as set forth below and reported herein refer to the amount, in grams, of an aqueous solution containing 0.9 weight percent sodium chloride a gram of the carboxyalkyl cellulose (carboxyalkyl polysaccharide) can absorb in 60 min under a load of 2.07 kPa (0.3 pounds per square inch). As a general rule, it is desired that the carboxyalkyl cellulose of the present invention have an Absorbency Under Load (AUL) of at least 17, beneficially of at least 20, most beneficially of at least 24, and preferably of at least 27 grams per gram.

Further, the carboxyalkyl cellulose of the present invention suitably has a Free-Swell Capacity of at least 20 grams, preferably of at least 30 grams, and most preferably of at least 35 grams. Free-Swell Capacity refers to the amount, in grams, of an aqueous solution containing 0.9 weight percent sodium chloride the carboxyalkyl cellulose can absorb in 60 min under no load. The exact procedure by which the Free-Swell Capacity is determined is set forth below in connection with the examples.

Any combination of time and temperature within the range of claim 1 which produces a crosslinked carboxyalkyl cellulose having the described Absorbency Under Load and Free-Swell capacity is preferred for use in the present invention. The inventors have found that there is generally an optimum combination of time and temperature at which to crosslink and optimize the Absorbency Under Load and Free-Swell Capacity of a particular carboxyalkyl cellulose material. If too little crosslinking occurs, the carboxyalkyl cellulose may possess a high Free-Swell Capacity but a relatively low Absorbency Under Load. If too much crosslinking occurs, the carboxyalkyl cellulose may have a relatively low Free-Swell Capacity and a relatively low Absorbency Under Load due to the inability of the carboxyalkyl cellulose to absorb much liquid.

The inventors found that solubilizing the carboxyalkyl cellulose in an aqueous solution and recovering prior to crosslinking produces a carboxyalkyl cellulose suitable for further crosslinking into materials having improved absorption properties. For example, a standard carboxymethyl cellulose heat-treated at 150°C for 60 min has an Absorbency Under Load of 6.4 grams and remains water soluble. When the same carboxymethyl cellulose is solubilized in water, recovered by evaporative drying and heat-treated at 150°C for 60 min, the carboxymethyl cellulose has an Absorbency Under Load of 24.8.

The inventors hypothesize that the solubilization may allow a molecular rearrangement of the carboxyalkyl cellulose which produces a more uniform distribution of the carboxyl groups and hydroxyl groups within the carboxyalkyl cellulose material. The more uniform distribution of the carboxyl groups within the carboxyalkyl cellulose may result in a more uniform crosslinking as a result of the heat-treatment step.

The inventors are uncertain as to whether or not the crosslinking which occurs is a chemical crosslinking, a physical crosslinking caused by the formation of crystal structures, or a combination of chemical and physical crosslinking. The exact cause of the improved absorbent properties achieved by the method of the present invention is not important so long as the improved absorbent properties are achieved.

In another aspect, the present invention relates to a waterswellable, water-insoluble carboxyalkyl polysaccharide produced by the method as set forth in the claims.

As described above, the carboxyalkyl polysaccharide is suitably a carboxyalkyl cellulose such as carboxymethyl cellulose, and carboxyethyl cellulose. The carboxyalkyl cellulose has an Absorbency Under Load value of at least 17, beneficially of at least 20, preferably of at least 24, and most preferably of at least 27. The carboxyalkyl cellulose has a Free-Swell Capacity of at least 20, beneficially of at least 30, and preferably of at least 35.

The carboxyalkyl polysaccharides of the present invention are suitable for use in personal care products such as diapers, training pants, feminine care products, adult incontinent products, wound dressings.

### Test Methods

### Absorbency Under Load

The Absorbency Under Load (AUL) is a test which measures the ability of an absorbent material to absorb a liquid (0.9 weight percent solution of sodium chloride in distilled water) while under an applied load or restraining force.

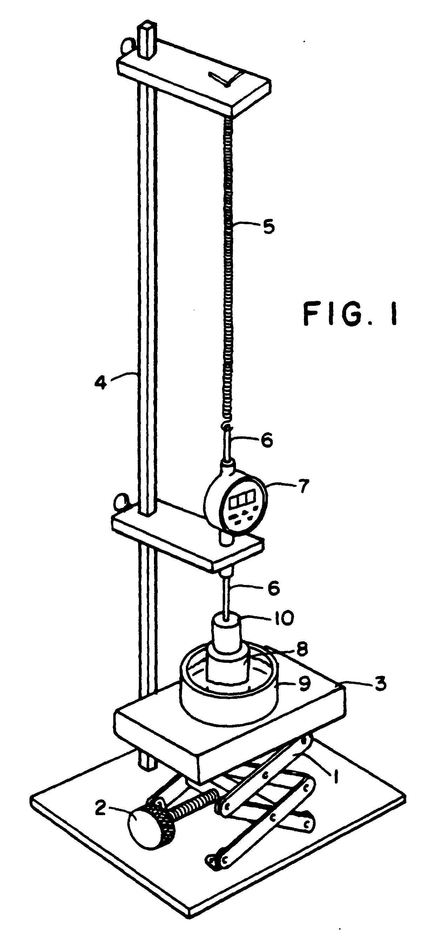

Referring to Fig. 1, the apparatus and method for determining AUL will be described. Shown is a perspective view of the apparatus in position during a test. Shown is a laboratory jack 1 having an adjustable knob 2 for raising and lowering the platform 3. A laboratory stand 4 supports a spring 5 connected to a modified thickness meter probe 6. which passes through the housing 7 of the meter, which is rigidly supported by the laboratory stand. A plastic sample cup 8, which contains the superabsorbent material sample to be tested, has a liquid-permeable bottom and rests within a Petri dish 9, which contains the saline solution to be absorbed. A weight 10 rests on top of a spacer disc (not visible) resting on top of the superabsorbent material sample (not visible).

The sample cup consists of a plastic cylinder having a 2.54 cm (1 inch) inside diameter and an outside diameter of 3.175 cm (1.25 inch). The bottom of the sample cup is formed by adhering a metal screen having 150 µm openings (100 mesh) to the end of the cylinder by heating the screen above the melting point of the plastic and pressing the plastic cylinder against the hot screen to melt the plastic and bond the screen to the plastic cylinder.

The modified thickness meter used to measure the expansion of the sample while absorbing the saline solution is a Mitutoyo Digimatic Indicator, IDC Series 543, Model 543-180, having a range of 0-1.27 cm (0-0.5 inch) and an accuracy of 0.001 mm (0.00005 inch) (Mitutoyo Corporation, 31-19, Shiba 5-chome, Minato-ku, Tokyo 108, Japan). As supplied from Mitutoyo Corporation, the thickness meter contains a spring attached to the probe within the meter housing. This spring is removed to provide a free falling probe, which has a downward force of about 27 grams. In addition, the cap over the top of the probe located on the top of the meter housing is also removed to enable attachment of the probe to the suspension spring 5 (available from McMaster-Carr Supply Co., Chicago, Illinois, Item No. 9640K41), which serves to counter or reduce the downward force of the probe to about 1 gram, ± 0.5 gram. A wire hook can be glued to the top of the probe for attachment to the suspension spring. The bottom tip of the probe is also provided with an extension needle (Mitutoyo Corporation, Part No. 131279) to enable the probe to be inserted into the sample cup.

To carry out the test, a 0.160 gram sample of the absorbent material, which has been sieved to a particle size between 300 and 600 µm, is placed into the sample cup. The sample is then covered with a plastic spacer disc, weighing 4.4 grams, which is slightly smaller than the inside diameter of the sample cup and serves to protect the sample from being disturbed during the test. The 100 gram weight is then placed on top of the spacer disc, thereby applying a load of 2.07 kPa (0.3 pounds per square inch). The sample cup is placed in the Petri dish on the platform of the laboratory jack raised up until it contacts the tip of the probe. The meter is zeroed. A sufficient amount of saline solution is added to the Petri dish (50-100 milliliters) to begin the test. The distance the weight is raised by the expanding sample as it absorbs the saline solution is measured by the probe. This distance, multiplied by the cross-sectional area inside the sample cup, is a measure of the expansion volume of the sample due to absorption. Factoring in the density of the saline solution and the weight of the sample, the amount of saline solution absorbed is readily calculated. The weight of saline solution absorbed after 60 min is the AUL value, expressed as grams saline solution absorbed per gram of absorbent. If desired, the readings of the modified thickness meter can be continuously input to a computer (Mitutoyo Digimatic Miniprocessor DP-2 DX) to make the calculations and provide AUL readings. As a cross-check, the AUL can also be determined by determining the weight difference between the sample cup before and after the test, the weight difference being the amount of solution absorbed by the sample.

### Free-Swell Capacity

The Free-Swell Capacity for a given absorbent material is determined in the same manner as the Absorbency Under Load, with the exception that the 100 gram weight is not placed on top of the spacer disc. The Free-Swell Capacity is reported as the weight of the saline solution absorbed after 60 min, expressed as grams of saline absorbed per gram of absorbent.

### Examples

### Example 1.

A sodium carboxymethyl cellulose commercially available from the Aqualon Company under the trade designation Aqualon™ Cellulose Gum CMC-7HCF or CMC-7H4F is provided. The carboxymethyl cellulose has an average degree of substitution of 0.7. CMC-7H4F has a slightly higher molecular weight than the CMC-7HCF. The carboxymethyl cellulose is dissolved in distilled water to form a solution containing 2 weight percent carboxymethyl cellulose based on total solution weight. The solution is then either left at a neutral pH, slightly acidified through the addition of hydrochloric acid (0.1 molar aqueous solution) or made basic (reference examples) by the addition of sodium hydroxide (0.1 molar aqueous solution). The carboxymethyl cellulose is recovered from the solution by evaporative drying at 80°C in a Blue M air-convection oven. After drying, the recovered carboxymethyl cellulose is ground into granules in a blender and heat-treated at various times and temperatures in an oven. Various combinations of temperature, time, and solution pH are made, and the physical properties of the resultant carboxymethyl cellulose determined. The exact process conditions and the physical properties of the resultant carboxymethyl cellulose are set forth in Table 1. Sample Nos. 1-38 employ the CMc-7HCF while Sample Nos. 39-45 employ the CMC-7H4F.

**TABLE 1**

| Sample No. | pH (DA)¹ | Treatment Temp (°C) | Treatment Time (min) | AUL² (g/g) | FSC³(g/g) |
|---|---|---|---|---|---|
| 1* | 6.08 (3) | N/A | N/A | 9.7 | |
| 2 | 6.08 (3) | 140 | 5 | 16.9 | |
| 3 | 6.08 (3) | 140 | 8 | 15.5 | |
| 4 | 6.08 (3) | 140 | 10 | 14.9 | |
| 5 | 6.08 (3) | 140 | 20 | 12.6 | |
| 6 | 6.08 (3) | 140 | 30 | 10.8 | |
| | | | | | |
| 7* | 6.44 (0.5) | N/A | N/A | 6.4 | |
| 8 | 6.44 (0.5) | 120 | 60 | 19.3 | |
| 9 | 6.44 (0.5) | 140 | 10 | 16.1 | |
| 10 | 6.44 (0.5) | 140 | 20 | 20.0 | |
| 11 | 6.44 (0.5) | 140 | 30 | 20.0 | |
| 12 | 6.44 (0.5) | 140 | 40 | 18.0 | |
| 13 | 6.44 (0.5) | 140 | 50 | 16.8 | |
| 14 | 6.44 (0.5) | 150 | 10 | 17.8 | |
| 15 | 6.44 (0.5) | 150 | 15 | 20.0 | |
| 16 | 6.44 (0.5) | 150 | 20 | 19.9 | |
| 17 | 6.44 (0.5) | 150 | 30 | 15.2 | |
| 18* | 7.4 (0) | N/A | N/A | 7.1 | |
| 19* | 7.4 (0) | 130 | 90 | 22.2 | |
| 20* | 7.4 (0) | 150 | 30 | 21.4 | 46.5 |
| 21* | 7.4 (0) | 150 | 40 | 25.3 | |
| 22* | 7.4 (0) | 150 | 45 | 27.1 | 44.6 |
| 23* | 7.4 (0) | 150 | 50 | 26.1 | |
| 24* | 7.4 (0) | 150 | 60 | 24.8 | 43.7 |
| 25* | 7.4 (0) | 150 | 75 | 23.7 | |
| 26* | 7.4 (0) | 150 | 90 | 21.6 | 34.8 |
| 27* | 7.4 (0) | 150 | 120 | 19.2 | 29.6 |
| | | | | | |
| 28* | 8.92 | N/A | N/A | 7.0 | |
| 29* | 8.92 | 150 | 40 | 17.1 | |
| 30* | 8.92 | 150 | 80 | 21.8 | |
| 31* | 8.92 | 150 | 100 | 20.9 | |
| 32* | 8.92 | 150 | 120 | 21.6 | |
| 33* | 8.92 | 150 | 150 | 18.6 | |
| 34* | 10.72 | N/A | N/A | 6.6 | |
| 35* | 10.72 | 150 | 50 | 11.6 | |
| 36* | 10.72 | 150 | 100 | 19.3 | |
| 37* | 10.72 | 150 | 150 | 20.4 | |
| 38* | 10.72 | 150 | 200 | 15.9 | |
| 39* | (0) | N/A | N/A | 8.1 | |
| 40 | (0) | 150 | 8 | 27.4 | |
| 41 | (0) | 150 | 10 | 27.7 | |
| 42 | (0) | 150 | 12 | 27.8 | |
| 43 | (0) | 150 | 15 | 27.0 | |
| 44 | (0) | 150 | 20 | 27.4 | |
| 45 | (0) | 150 | 60 | 18.5 | |

| | | | | | |
|---|---|---|---|---|---|
| N/A = Not applicable * Not an example of the present invention | | | | | |
| ¹ pH of the solution of carboxymethyl cellulose and water prior to recovery (Degree of Molar Acidification) | | | | | |
| ² Absorbency Under Load in grams absorbed aqueous saline solution (0.9 weight percent) per gram of carboxymethyl cellulose under a load of 2.07 kPa (0.3 psi). | | | | | |
| ³ Free Swell Capacity in grams absorbed aqueous saline solution (0.9 weight percent) per gram of carboxymethyl cellulose. | | | | | |

### Example 2.

Example 1 is repeated with the exception that a sodium carboxymethyl cellulose commercially available from the Aqualon Company under the trade designation Aqualon @ Cellulose Gum CMC-9H4 is employed. The carboxymethyl cellulose has an average degree of substitution of 0.9. Again, the exact process conditions and physical properties of the resultant carboxymethyl cellulose are set forth in Table 2.

**TABLE 2**

| Sample No. | DA (mol%)¹ | Treatment Temp (°C) | Treatment Time (min) | AUL² (g/g) | FSC³(g/g) |
|---|---|---|---|---|---|
| 46 | 3 | 150 | 30 | 10.5 | |
| 47* | 0.5 | N/A | N/A | 7.2 | |
| 48 | 0.5 | 150 | 5 | 17.9 | |
| 49 | 0.5 | 150 | 8 | 22 | |
| 50 | 0.5 | 150 | 10 | 22.3 | |
| 51 | 0.5 | 150 | 15 | 20.5 | |
| 52 | 0.5 | 150 | 20 | 18.3 | |
| 53 | 0.5 | 150 | 30 | 16 | |
| 54* | 0.1 | N/A | N/A | 6.9 | |
| 55 | 0.1 | 150 | 10 | 10.7 | |
| 56 | 0.1 | 150 | 20 | 22.1 | |
| 57 | 0.1 | 150 | 30 | 22.2 | |
| 58 | 0.1 | 150 | 40 | 20.4 | |
| 59 | 0.1 | 150 | 60 | 18.8 | |
| 60* | 0 | N/A | N/A | 7.4 | |
| 61 | 0 | 130 | 60 | 8.7 | |
| 62 | 0 | 130 | 90 | 15.8 | |
| 63 | 0 | 130 | 120 | 20.3 | |
| 64 | 0 | 130 | 150 | 22.8 | |
| 65* | 0 | N/A | N/A | 7.4 | |
| 66 | 0 | 170 | 10 | 19.8 | |
| 67 | 0 | 170 | 20 | 13.3 | |
| 68 | 0 | 170 | 30 | 10.5 | |
| N/A = Not Applicable | | | | | |

| | | | | | |
|---|---|---|---|---|---|
| * Not an example of the present invention | | | | | |
| ¹ Degree of Molar Acidilication | | | | | |
| ² Absorbency Under Load in grams absorbed aqueous saline solution (0.9 weight percent) per gram of carboxymethyl cellulose under a load of 0.3 psi. | | | | | |
| ³ Free Swell Capacity in grams absorbed aqueous saline solution (0.9 weight percent) per gram of carboxymethyl cellulose. | | | | | |

### Example 3

Example 2 is repeated with the exception that the carboxymethyl cellulose is recovered and comminuted to form both granules and flakes. The carboxymethyl cellulose is then heat-treated at 150°C for various times to determine the effect of geometry on the heat-treatment process. The results are set forth in Table 3. Samples 69-73 are in granular form. Samples 74-80 are in the form of flakes.

**TABLE 3**

| Sample No. | DA (mol%)¹ | Treatment Temp (°C) | Treatment Time (min) | AUL² (g/g) |
|---|---|---|---|---|
| 69* | 0 | N/A | N/A | 7.4 |
| 70 | 0 | 150 | 20 | 19 |
| 71 | 0 | 150 | 30 | 24 |
| 72 | 0 | 150 | 40 | 24.5 |
| 73 | 0 | 150 | 50 | 22.4 |
| 74* | 0 | N/A | N/A | 7.4 |
| 75 | 0 | 150 | 10 | 9.8 |
| 76 | 0 | 150 | 15 | 15.8 |
| 77 | 0 | 150 | 20 | 25 |
| 78 | 0 | 150 | 30 | 23 |
| 79 | 0 | 150 | 45 | 20.8 |
| 80 | 0 | 150 | 60 | 18.4 |
| N/A = Not Applicable | | | | |

| | | | | |
|---|---|---|---|---|
| * Not an example of the present invention | | | | |
| ¹ Degree of Molar Acidification | | | | |
| ² Absorbency Under Load in grams absorbed aqueous saline solution (0.9 weight percent) per gram of carboxymethyl cellulose under a load of 0.3 psi. | | | | |

### Example 4

Example 2 is repeated with the exception that the carboxymethyl cellulose is dissolved in distilled water to form a solution containing 23 weight percent carboxymethyl cellulose based on total solution weight. The recovered carboxymethyl cellulose is heat-treated in the form of granules at 150°C for various time periods. The physical properties of the resultant polymer are set forth in Table 4.

**TABLE 4**

| Sample No. | DA (mol%)¹ | Treatment Temp (°C) | Treatment Time (min) | AUL² (g/g) |
|---|---|---|---|---|
| 81* | 0 | N/A | N/A | 7.3 |
| 82 | 0 | 150 | 20 | 22 |
| 83 | 0 | 150 | 30 | 22.2 |
| 84 | 0 | 150 | 40 | 20.5 |
| N/A = Not Applicable | | | | |

| | | | | |
|---|---|---|---|---|
| * Not an example of the present invention | | | | |
| ¹ Degree of Molar Acidilication | | | | |
| ² Absorbency Under Load in grams absorbed aqueous saline solution (0.9 weight percent) per gram of carboxymethyl cellulose under a load of 0.3 psi. | | | | |

Control samples of the carboxymethyl cellulose of Example 1 and Example 2 in which the carboxymethyl cellulose is subjected to a heat-treatment process without having been dissolved in an aqueous solution and recovered were performed. Again, the exact process conditions of the heat-treatment step and the physical properties of the resultant polymer are set forth in Table 5. Both control samples remain water soluble even after the heat-treatment step.

**TABLE 5**

| Sample No. | Treatment Temp (°C) | Treatment Time (min) | AUL(g/g) |
|---|---|---|---|
| 85* | 150 | 15 | 6.8 |
| 86* | 150 | 60 | 6.4 |

| | | | |
|---|---|---|---|
| * Not an example of the present invention | | | |

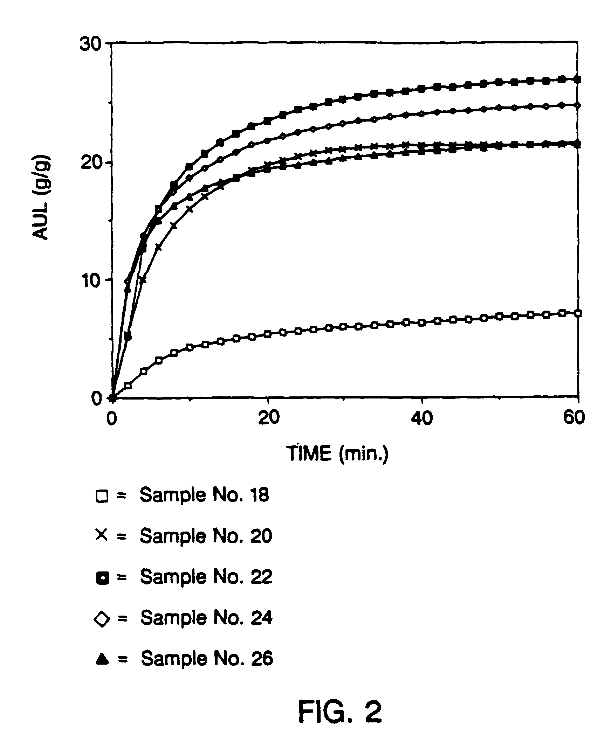

Fig. 2 illustrates the Absorbency Under Load of the carboxymethyl cellulose of Example 1 heat-treated at a temperature of 150°C for various lengths of time. It is noted that the optimum heat-treatment time appears to be approximately 45 min, producing an absorbent carboxymethyl cellulose having an Absorbency Under Load of about 27.

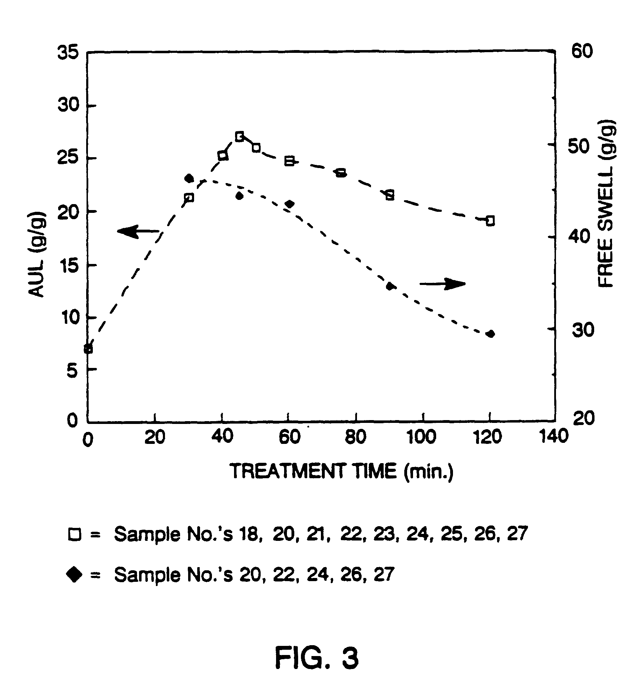

Fig. 3 illustrates the effect of time of heat-treatment on Absorbency Under Load values and Free-Swell Capacity as a function of treatment time at 150°C. As can be seen from reference to Fig. 3, an optimum treatment time can be determined.

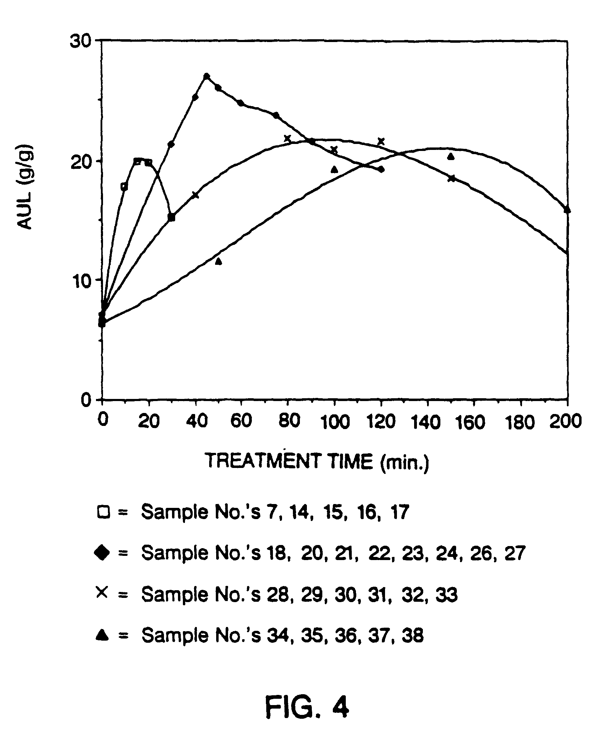

Fig. 4 illustrates the effect of the initial pH of the solution of carboxymethyl cellulose and water on the Absorbency Under Load of carboxymethyl celluloses treated at 150°C for various periods of time. As can be seen from reference to Fig. 4, varying the initial pH of the solution of carboxymethyl cellulose and water alters the optimum treatment time. As a general rule, the lower the pH, the shorter the treatment time to optimize absorbent properties.

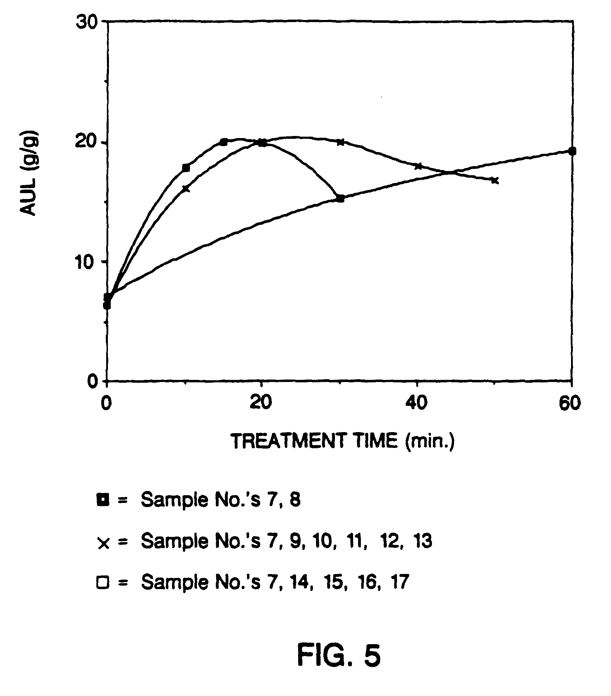

Fig. 5 shows the effect of temperature of heat-treatment on the Absorbency Under Load values of carboxymethyl cellulose of a slightly acidified system (0.5 DA). As can be seen from reference to Fig. 5, the higher the treatment temperature, the shorter the time necessary to obtain the optimum Absorbency Under Load value.

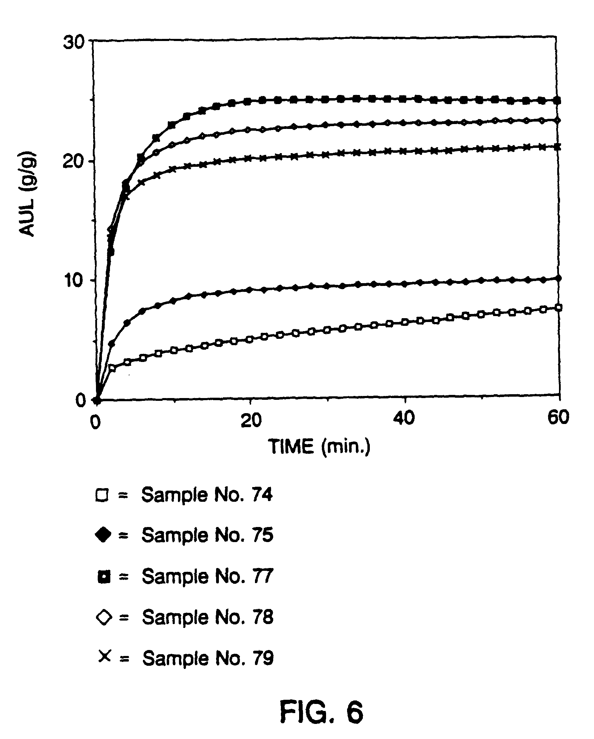

Fig. 6 illustrates the Absorbency Under Load value of the carboxymethyl cellulose of Example 3 heat-treated at 150°C for various times. Again, as can be seen from reference to Fig. 6, an optimum heat-treatment time can be determined. In the case of Fig. 6, the optimum time is about 20 minutes.

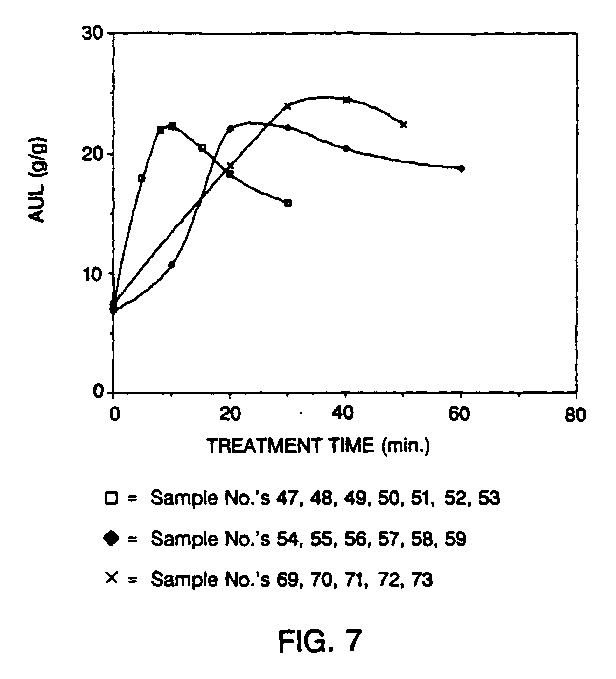

Fig. 7 illustrates the effect of the degree of molar acidification on the carboxymethyl cellulose of Examples 2 and 3. As can be seen from reference to Fig. 7, the more acidic the carboxymethyl cellulose, the shorter the time necessary to achieve the optimum Absorbency Under Load value.

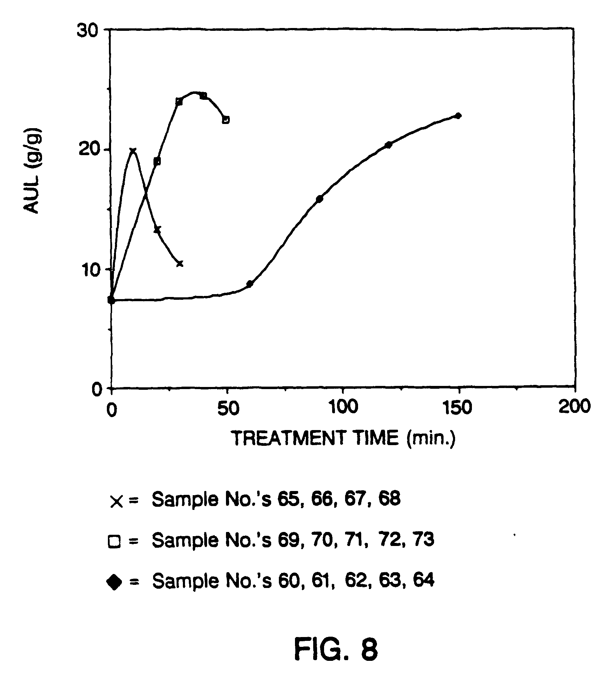

Fig. 8 illustrates the effect of temperature of the heat-treatment process on the Absorbency Under Load performance of the carboxymethyl cellulose of Examples 2 and 3. Again, it is seen that as the temperature of heat-treatment increases, the time necessary to achieve to the optimum Absorbency Under Load value decreases.

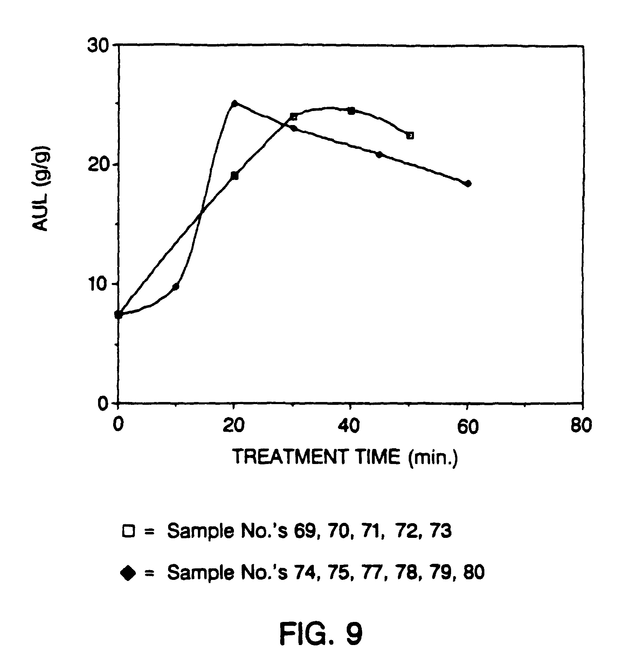

Fig. 9 illustrates the effect of the geometry of the carboxymethyl cellulose on the Absorbency Under Load performance. As can be seen from reference to Fig. 9, carboxymethyl cellulose in the form of flakes tends to reach the optimum Absorbency Under Load value faster.

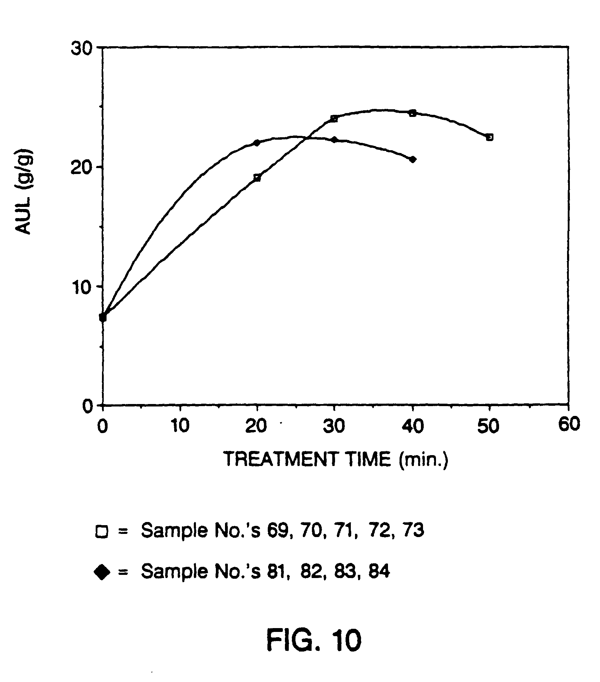

Fig. 10 illustrates the effect of the concentration of carboxymethyl cellulose in the initial solution of carboxymethyl cellulose and water. Reference to Fig. 10 shows that, at higher concentrations of carboxymethyl cellulose, lower maximum Absorbency Under Load values are achieved, but that such values are achieved faster than at lower concentrations.

Accordingly, the specific examples set forth above are not intended to limit, in any manner, the scope of the invention as set forth in the appended claims.

## Claims

1. A method for producing a water-swellable, water-insoluble carboxyalkyl polysaccharide, **characterized by** the following steps:
forming a solution consisting of a water-soluble carboxyalkyl polysaccharide and water as the solvent and optionally an acid wherein said solution is acidic or neutral, wherein said water-soluble carboxyalkyl polysaccharide dissolves into said water, said carboxyalkyl polysaccharide having an average degree of substitution from 0.3 to 1.5;
recovering said carboxyalkyl polysaccharide from said solution by evaporative drying; and
heat-treating said recovered carboxyalkyl polysaccharide at a temperature of from 120°C to 200°C and for a time of from 1 min. to 120 min. to crosslink said carboxyalkyl polysaccharide to render said carboxyalkyl polysaccharide water insoluble, wherein said water-insoluble carboxyalkyl polysaccharide has an Absorbency Under Load, as defined in the description, of at least 17 (grams/gram) and a Free-Swell Capacity, as defined in the description, of at least 20 (grams/gram).

2. The method according to claim 1 wherein the carboxyalkyl polysaccharide is a carboxyalkyl cellulose.

3. The method according to claim 1 or 2, wherein the solution comprises from 0.01 to 90 weight percent of said carboxyalkyl polysaccharide.

4. The method according to claim 2 or 3, wherein said carboxyalkyl cellulose has a degree of substitution of from 0.4 to 1.2.

5. The method according to any of claims 1 to 4, particularly of claim 2, wherein said carboxyalkyl cellulose is carboxymethyl cellulose.

6. The method according to any of claims 2 to 5, particularly of claim 2, wherein said recovered carboxyalkyl cellulose is heat-treated at a temperature and for a time sufficient to provide said carboxyalkyl cellulose with an Absorbency Under Load, as defined in the description, of at least 20.

7. The method according to claim 6, wherein said Absorbency Under Load, as defined in the description, is at least 24.

8. The method according to claim 6, wherein said Absorbency Under Load, as defined in the description, is at least 27.

9. The method according to claim 1, wherein said carboxyalkyl cellulose is heat-treated at a temperature of from 130°C to 170°C for a time of from 1 min. to 120 min.

10. The method according to claim 9, wherein said carboxyalkyl cellulose is heat-treated at a temperature of from 130°C to 170°C for a time of from 5 min. to 60 min.

11. The method according to any of claims 1 to 10, further comprising the step of comminuting said carboxyalkyl polysaccharide after recovery and before heat-treating.

12. A method for producing a water-swellable, water-insoluble carboxyalkyl polysaccharide, **characterized by** the following sequential steps:
forming a solution consisting of from 0.01 to 90 weight percent of a water-soluble carboxyalkyl polysaccharide based on a total solution weight and water as the solvent and optionally an acid wherein said solution is acidic or neutral, wherein said water-soluble carboxyalkyl polysaccharide dissolves into said water, said carboxyalkyl polysaccharide having an average degree of substitution of from 0.4 to 1.2;
recovering said carboxyalkyl polysaccharide from said solution by evaporative drying; and
heat-treating said recovered carboxyalkyl polysaccharide at a temperature of from 120°C to 200°C for a time of from 1 min. to 120 min., such that said carboxyalkyl polysaccharide is rendered generally water-insoluble and has an Absorbency Under Load, as defined in the description, of at least 17 (grams/gram) and a Free-Swell Capacity, as defined in the description, of at least 20 (grams/gram).

13. The method according to claim 12 wherein the carboxyalkyl polysaccharide is a carboxyalkyl cellulose.

14. A carboxyalkyl polysaccharide produced by the method of any of claims 1 to 13.

15. Use of a carboxyalkyl polysaccharide according to claim 14 for producing absorbent products.

## Patentansprüche

1. Verfahren zur Herstellung eines wasserquellbaren, wasserunlöslichen Carboxyalkylpolysaccharids, **gekennzeichnet durch** folgende Schritte:
Bildung einer Lösung mit einem wasserlöslichen Carboxyalkylpolysaccharid und Wasser als Lösungsmittel, und wahlweise einer Säure, wobei die Lösung sauer oder neutral ist, wobei sich das wasserlösliche Carboxyalkylpolysaccharid in dem Wasser auflöst, wobei das Carboxyalkylpolysaccharid einen durchschnittlichen Substitutionsgrad von 0,3 bis 1,5 aufweist;
Rückgewinnung des Carboxyalkylpolysaccharids aus der Lösung **durch** Verdunstungstrocknung; und
Wärmebehandlung des gewonnenen Carboxyalkylpolysaccharids bei einer Temperatur von 120°C bis 200°C und über eine Zeitdauer von 1 min bis 120 min, um das Carboxyalkylpolysaccharid zu vernetzen, um das Carboxyalkylpolysaccharid wasserunlöslich zu machen, wobei das wasserunlösliche Carboxyalkylpolysaccharid ein Absorptionsvermögen unter Belastung, wie in der Beschreibung definiert, von mindestens 17 (Gramm/Gramm) und eine Freiquellkapazität, wie in der Beschreibung definiert, von mindestens 20 (Gramm/Gramm) aufweist.

2. Verfahren gemäß Anspruch 1, bei dem das Carboxyalkylpolysaccharid eine Carboxyalkylcellulose ist.

3. Verfahren gemäß Anspruch 1 oder 2, bei dem die Lösung 0,01 bis 90 Gew.% des Carboxyalkylpolysaccharids enthält.

4. Verfahren gemäß Anspruch 2 oder 3, bei dem die Carboxyalkylcellulose einen Substitutionsgrad von 0,4 bis 1,2 aufweist.

5. Verfahren gemäß einem der Ansprüche 1 bis 4, insbesondere gemäß Anspruch 2, bei dem die Carboxyalkylcellulose Carboxymethylcellulose ist.

6. Verfahren gemäß einem der Ansprüche 2 bis 5, insbesondere gemäß Anspruch 2, bei dem die rückgewonnene Carboxyalkylcellulose bei einer Temperatur und über eine Zeitdauer wärmebehandelt wird, welche ausreichen, um der Carboxyalkylcellulose ein Absorptionsvermögen unter Belastung, wie in der Beschreibung definiert, von mindestens 20 zu verleihen.

7. Verfahren gemäß Anspruch 6, bei dem das Absorptionsvermögen unter Belastung, wie in der Beschreibung definiert, mindestens 24 beträgt.

8. Verfahren gemäß Anspruch 6, bei dem das Absorptionsvermögen unter Belastung, wie in der Beschreibung definiert, mindestens 27 beträgt.

9. Verfahren gemäß Anspruch 1, bei dem die Carboxyalkylcellulose bei einer Temperatur von 130°C bis 170°C über eine Zeitdauer von 1 Minute bis 120 Minuten wärmebehandelt wird.

10. Verfahren gemäß Anspruch 9, bei dem die Carboxyalkylcellulose bei einer Temperatur von 130°C bis 170°C über eine Zeitdauer von 5 Minuten bis 60 Minuten wärmebehandelt wird.

11. Verfahren gemäß einem der Ansprüche 1 bis 10 mit dem weiteren Schritt der Zerkleinerung des Carboxyalkylpolysaccharids nach der Gewinnung und vor der Wärmebehandlung.

12. Verfahren zur Herstellung eines wasserquellbaren, wasserunlöslichen Carboxyalkylpolysaccharids, **gekennzeichnet durch** die folgenden aufeinanderfolgenden Schritte:
Bildung einer Lösung mit 0,01 bis 90 Gew.% eines wasserlöslichen Carboxyalkylpolysaccharids, bezogen auf das Gesamtlösungsgewicht, und Wasser als Lösungsmittel, und wahlweise einer Säure, wobei die Lösung sauer oder neutral ist, wobei sich das wasserlösliche Carboxyalkylpolysaccharid in dem Wasser auflöst, wobei das Carboxyalkylpolysaccharid einen durchschnittlichen Substitutionsgrad von 0,4 bis 1,2 aufweist;
Rückgewinnung des Carboxyalkylpolysaccharids aus der Lösung **durch** Verdunstungstrocknung; und
Wärmebehandlung des gewonnenen Carboxyalkylpolysaccharids bei einer Temperatur von 120°C bis 200°C über eine Zeitdauer von 1 Minute bis 120 Minuten, so daß das Carboxyalkylpolysaccharid im allgemeinen wasserunlöslich wird und ein Absorptionsvermögen unter Belastung, wie in der Beschreibung definiert, von mindestens 17 (Gramm/Gramm) und eine Freiquellkapazität, wie in der Beschreibung definiert, von mindestens 20 (Gramm/Gramm) aufweist.

13. Verfahren gemäß Anspruch 12, bei dem das Carboxyalkylpolysaccharid eine Carboxyalkylcellulose ist.

14. Carboxyalkylpolysaccharid, hergestellt durch das Verfahren gemäß einem der Ansprüche 1 bis 13.

15. Verwendung eines Carboxyalkylpolysaccharids gemäß Anspruch 14 zur Herstellung von saugfähigen Produkten.

## Revendications

1. Procédé de production d'un carboxyalkyl-polysaccharide hydroinsoluble et gonflable dans l'eau, **caractérisé par** les étapes suivantes :
la formation d'une solution constituée d'un carboxyalkyl-polysaccharide hydrosoluble et d'eau comme solvant, et facultativement d'un acide, ladite solution étant acide ou neutre, ledit carboxyalkyl-polysaccharide hydrosoluble se dissolvant dans ladite eau et ledit carboxyalkyl-polysaccharide ayant un degré moyen de substitution compris entre 0,3 et 1,5 ;
la récupération dudit carboxyalkyl-polysaccharide depuis ladite solution par séchage par évaporation ; et
le traitement thermique dudit carboxyalkyl-polysaccharide récupéré à une température comprise entre 120°C et 200°C et pendant une durée allant de 1 minute à 120 minutes pour réticuler ledit carboxyalkyl-polysaccharide aux fins de le rendre hydroinsoluble, ledit carboxyalkyl-polysaccharide hydroinsoluble ayant une Capacité d'Absorption sous Charge, telle que définie dans la description, d'au moins 17 (grammes/gramme) et une Capacité de Gonflement Libre, telle que définie dans la description, d'au moins 20 (grammes/gramme).

2. Procédé selon la revendication 1, dans lequel le carboxyalkyl-polysaccharide est une carboxyalkyl-cellulose.

3. Procédé selon la revendication 1 ou 2, dans lequel la solution comprend de 0,01% à 90% en poids dudit carboxyalkyl-polysaccharide.

4. Procédé selon la revendication 2 ou 3, dans lequel ladite carboxyalkyl-cellulose a un degré de substitution allant de 0,4 à 1,2.

5. Procédé selon l'une quelconque des revendications 1 à 4, en particulier selon la revendication 2 dans lequel ladite carboxyalkyl-cellulose est la carboxyméthylcellulose.

6. Procédé selon l'une quelconque des revendications 2 à 5, en particulier selon la revendication 2, dans lequel ladite carboxyalkyl-cellulose récupérée est traitée thermiquement à une température et pendant une durée suffisantes pour donner à ladite carboxyalkyl-cellulose une Capacité d'Absorption sous Charge, telle que définie dans la description, d'au moins 20.

7. Procédé selon la revendication 6, dans lequel ladite Capacité d'Absorption sous Charge, telle que définie dans la description, est d'au moins 24.

8. Procédé selon la revendication 6, dans lequel ladite Capacité d'Absorption sous Charge, telle que définie dans la description, est d'au moins 27.

9. Procédé selon la revendication 1, dans lequel ladite carboxyalkyl-cellulose est traitée thermiquement à une température comprise entre 130°C et 170°C pendant une durée allant d'une minute à 120 minutes.

10. Procédé selon la revendication 9, dans lequel ladite carboxyalkyl-cellulose est traitée thermiquement à une température comprise entre 130°C et 170°C pendant une durée comprise entre 5 minutes et 60 minutes.

11. Procédé selon l'une quelconque des revendications 1 à 10, comprenant en outre l'étape de réduction en particules dudit carboxyalkyl-polysaccharide après récupération et avant traitement thermique.

12. Procédé de production d'un carboxyalkyl-polysaccharide hydroinsoluble et gonflable dans l'eau, **caractérisé par** las étapes successives suivantes :
la formation d'une solution constituée de 0,01 à 90% en poids d'un carboxyalkyl-polyaaccharide hydrosoluble, par rapport au poids total de la solution, et d'eau comme solvant, et facultativement d'un acide, ladite solution étant acide ou neutre, ledit carboxyalkyl-polysaccharide hydrosoluble se dissolvant dans ladite eau, ledit carboxyalkyl-polysaccharide ayant un degré moyen de substitution compris entre 0,4 et 1,2 ;
la récupération dudit carboxyalkyl-polysaccharide à partir de ladite solution par séchage par évaporation ; et
le traitement thermique dudit carboxyalkyl-polysaccharide récupéré à une température comprise entre 120°C et 200°C pendant une durée comprise entre 1 minute et 120 minutes, de telle sorte que ledit carboxyalkyl-polysaccharide est rendu généralement hydroinaoluble et a une Capacité d'Absorption sous Charge, telle que définie dans la description, d'au moins 17 (grammes/gramme) et une Capacité de Gonflement Libre, telle que définie dans la description, d'au moins 20 (grammes/gramme).

13. Procédé selon la revendication 12, dans lequel le carboxyalkyl-polysaccharide est une carboxyalkyl-cellulose.

14. Carboxyalkyl-polysaccharide produit par le procédé selon l'une quelconque des revendications 1 à 13.

15. Utilisation d'un carboxyalkyl-polysaccharide selon la revendication 14 dans la production de produits absorbants.
